Europäisches Patentamt

European Patent Office

Office européen des brevets

(19)

(11) Veröffentlichungsnummer: **0 021 245**
**B1**

(12) ## EUROPÄISCHE PATENTSCHRIFT

(45) Veröffentlichungstag der Patentschrift:
**27.04.83**

(21) Anmeldenummer: **80103245.9**

(22) Anmeldetag: **10.06.80**

(51) Int. Cl.³: **G 01 N 33/82**, G 01 N 33/14

(54) Schnelltest zum Nachweis von Ascorbinsäure und Verfahren zu dessen Herstellung.

(30) Priorität: **28.06.79 DE 2926068**

(43) Veröffentlichungstag der Anmeldung:
**07.01.81 Patentblatt 81/1**

(45) Bekanntmachung des Hinweises auf die Patenterteilung:
**27.04.83 Patentblatt 83/17**

(84) Benannte Vertragsstaaten:
**AT BE CH DE FR GB IT LI LU NL SE**

(56) Entgegenhaltungen:
**DE-A-2 309 794**

(73) Patentinhaber: **BOEHRINGER MANNHEIM GMBH,
Sandhofer Strasse 112-132 Postfach 31 01 20,
D-6800 Mannheim 31-Waldhof (DE)**

(72) Erfinder: **Bleisteiner, Manfred, Feldstrasse 93,
D-6800 Mannheim 51 (DE)**
Erfinder: **Rittersdorf, Walter, Dr.rer.nat., Kasseler
Strasse 6, D-6800 Mannheim 31 (DE)**
Erfinder: **Wielinger, Hans, Dr.phil., Kronberger
Strasse 14, D-6800 Mannheim 31 (DE)**

Schnelltest zum Nachweis von Ascorbinsäure und Verfahren zu dessen Herstellung

Die Erfindung betrifft einen stabilen Schnelltest zum semiquantitativen Nachweis von Ascorbinsäure in Getränken und Körperflüssigkeiten.

Vor allem in Jahreszeiten, in denen witterungsbedingt viele Erkältungskrankheiten auftreten, werden zu therapeutischen Zwecken und zur Vorbeugung hohe Dosen Vitamin C gegeben. Die Mengen an Ascorbinsäure, die im Körper nicht verstoffwechselt werden, werden vor allem über den Harn ausgeschieden; dabei sind häufig Konzentrationen von 20 mg/dl und mehr zu beobachten. 20 mg/dl Harn ist ungefähr die Grenzkonzentration, ab der die Nachweisreaktionen anderer für die Diagnose wichtiger Parameter, wie z.B. Glucose, Blut usw., von Ascorbinsäure so stark gestört werden, dass eine Fehldiagnose nicht auszuschliessen ist. Für den Arzt ist es daher von grosser Bedeutung, über das Vorhandensein und die Menge dieser reduzierenden Störsubstanz genau informiert zu sein.

Auch in der Getränkeindustrie ist es wichtig zu wissen, wieviel Ascorbinsäure in Fruchtsäften, Weinen usw. enthalten sind. Der Gehalt an Vitamin C ist mitunter ein wesentliches Kriterium für die Qualität des Produktes.

Zum Nachweis von Ascorbinsäure sind verschiedene nasschemische Verfahren bekannt (Römpps Chemie-Lexikon, 7. Auflage, Franckh'sche Verlagsbuchhandlung, Stuttgart 1972, Seite 3835), die z.B. auf der Reduktion bestimmter Farbstoffe aufbauen [Entfärbung von Dichlorphenolindophenol (Tillmans Reagenz), Methylenblau, Jod], das chromatographische Verhalten im Dünnschicht- oder Papierchromatogramm benutzen oder spezielle Reaktionen, wie die Dehydrierung zur Dehydroascorbinsäure und deren Überführung in das farbige Osazon mit 2,4-Dinitropenylhydrazin ausnutzen.

Die nasschemischen Verfahren haben sich wegen des notwendigen apparativen und zeitlichen Aufwandes nicht immer bewährt und werden insbesondere für qualitative und semiquantitative Proben von Schnelltesten verdrängt.

Die bisher bekanntgewordenen Schnellteste zur Bestimmung von Ascorbinsäure basieren grundsätzlich auf zwei bereits aus der «Nasschemie» bekannten Verfahren:

a) Entfärbung bestimmter Farbstoffe durch Reduktion (DE-OS 2 835 743).

b) Erzeugung einer Färbung durch Reduktion von Phosphormolybdaten zu Molybdänblau (DE-AS 2 309 794).

Schnellteste, die auf einer Entfärbungsreaktion beruhen, haben den prinzipiellen Nachteil, dass reproduzierbare Testergebnisse nur dann erhalten werden, wenn der Farbstoff exakt dosiert wird und absolut stabil ist. Es liegt auf der Hand, dass die Produktion reproduzierbarer Chargen solcher Teststreifen erhebliche Ansprüche an die Fertigung und die Qualitätskontrolle stellen. Weiterhin haben diese Teste den Nachteil, dass die relativ stärksten Konzentrationsänderungen im unteren Konzentrationsbereich des Substrats die relativ geringsten Konzentrationsänderungen des Farbstoffes bewirken.

In Spot Tests in Org. Analysis, 1960, S. 405, ist ein Schnelltest beschrieben, der auf der Reduktion von Ammoniumphosphormolybdat beruht. Der Test hat sich jedoch für die Praxis nicht bewährt, da er einerseits durch eine umständliche Doppelimprägnierung hergestellt werden muss und andererseits nur einige Tage stabil ist. Darüber hinaus ist es notwendig, die Proben vor der Untersuchung anzusäuern, da der Test im neutralen und alkalischen Medium durch andere Stoffe gestört wird.

Als Verbesserung dieses Testes wird in der DE-AS 2 309 794 ein Schnelltest, bestehend aus einem Gemisch aus 2,18- und 2,24-Phosphormolybdatsäure, einem neutralen Nitrat und einer organischen Säure, vorzugsweise Malonsäure, vorgeschlagen. Dieser an sich brauchbare Schnelltest hat jedoch immer noch folgende Nachteile:

— Die Verwendung eines Gemisches aus zwei chemisch sehr ähnlichen Heteropolysäuren stellt erhebliche Anforderungen an die Qualitätskontrolle,

— die bevorzugte Malonsäure und viele andere organische Säuren haben die Eigenschaft, selbst bei Raumtemperatur bei längerer Lagerung im geschlossenen Gefäss aus dem Testbezirk teilweise abzusublimieren. Das hat zur Folge, dass in Kombinationsteststreifen während Lagerung und Transport andere Testbezirke negativ beeinflusst werden. So wird z.B. eine pH-Anzeige mit dem entsprechenden Reagenzpapier verfälscht.

Es bestand daher die Aufgabe, einen Schnelltest für Ascorbinsäure zu entwickeln, der die oben genannten Nachteile nicht aufweist, d.h., der

— auf dem bewährten Prinzip der Färbung beruht

— Ascorbinsäure in Fruchtsäften (20-300 mg%) und Körperflüssigkeiten (0-50 mg%) gleich gut nachweist

— einfach und billig bezüglich Herstellung und Qualitätskontrolle ist und

— sich auch für den Einsatz in Mehrfachtesten, insbesondere zusammen mit einem pH-Test eignet.

Es hat sich nun überraschenderweise gezeigt, dass man Schnellteste gemäss obiger Aufgabenstellung erhält, die mit leuchtend blauen, gut abgestuften Farben reagieren, wenn man folgende Bestandteile verwendet:

— Alkalisalz der 2,18-Phosphormolybdänsäure

— eine aliphatische Hydroxycarbonsäure, ggf. im Gemisch mit ihrem Alkalisalz

— ein Alkalichlorat.

Die Alkalisalze der 2,18-Phosphormolybdänsäure sind gutbekannte Verbindungen [A. Rosenheim u. A. Traube, Z. anorg. Chem. *65* 96-101 (1910)]. Unter aliphatischen Hydroxycarbonsäuren werden solche Säuren verstanden, die neben der oder den -COOH-Gruppen noch eine oder mehrere OH-Gruppen im Molekül enthalten. Es wurde festgestellt, dass solche Verbindungen auch bei längerer Lagerung im geschlossenen Gefäss nicht sublimieren und so andere Testbezirke beeinflussen. Besonders bevorzugte Hydroxycarbonsäuren sind die handelsüblichen, preiswerten Äpfel-, Citronen- und Weinsäuren.

Auch die Alkalichlorate sind bekannte, handelsübliche Verbindungen. Das Alkalichlorat dient zur Stabilisierung des Testpapiers, da es eine Bläuung durch reduzierende Bestandteile des Papiers während der Lagerung verhindert. Es muss als überraschend angesehen werden, dass das starke Oxidationsmittel Chlorat zwar die Reduktion der Molybdate bei der Lagerung verhindert, aber die analoge Reduktion durch Ascorbinsäure beim Rest nicht beeinflusst.

Die erfindungsgemässen Testpapiere werden beispielsweise wie folgt hergestellt:

Das Alkalisalz der 2,18-Phosphormolybdänsäure in einer Konzentration von 3,0-9,0 g/l wird zusammen mit 2,0-6,0 g/l Alkalichlorat und 10-90 g/l einer aliphatischen Hydroxycarbonsäure in Wasser mit oder ohne Zusatz eines beliebigen mit Wasser mischbaren organischen Lösungsmittel bzw. eines niederen Alkohols gelöst. Mit einer Alkalilauge, wie Lithiumhydroxyd, Natriumhydroxyd usw. wird die Lösung auf einen pH von 2,5-5,0 gebracht.

Selbstverständlich kann man auch so vorgehen, dass man ein Gemisch aus der Hydroxycarbonsäure und dem entsprechenden Alkalisalz einsetzt, wobei das Mischungsverhältnis so gewählt wird, dass ein pH-Wert der Imprägnierlösung von 2,5-5,0 erhalten wird. Auch Gemische der o.g. Säuren können eingesetzt werden. Mit der so hergestellten Tränklösung werden nach üblichem Verfahren Filterpapiere imprägniert. Nach dem Trocknen werden die Reagenzpapiere geschnitten und z.B. durch Aufkleben auf einen Handgriff aus Kunststoff-Folie oder Einnetzen gemäss DAS 2 118 455 zu Einzel- oder Kombinationsteststreifen weiter verarbeitet. Mit dem so hergestellten Teststreifen kann der Ascorbinsäure-Gehalt in Flüssigkeiten entweder anhand von Vergleichsfarbentafeln oder mit Hilfe remissionsphotometrischer Verfahren bestimmt werden.

Die Erfindung wird durch folgende Beispiele näher erläutert:

*Beispiel 1*

7 g Natriumsalz der 2,18-Phosphormolybdänsäure, 4 g Natriumchlorat, 40 g Citronensäure und 13,5 g tri-Lithiumcitrat werden in einem Gemisch aus 600 ml Methanol und 380 ml Wasser gelöst. Mit dieser Lösung, die einen pH-Wert von 3,6 aufweist, wird ein Filterpapier z.B. Typ 2668 der Firma Schleicher + Schüll imprägniert und 1 Stunde bei 60°C getrocknet. Aus dem so hergestellten Reagenzpapier werden 6 × 6 mm grosse Stücke geschnitten und zwischen einen Kunststoffhandgriff und einem dünnen Nylonnetz gemäss DE-AS 2 118 455 eingesiegelt. Diese Teststreifen geben in Harnen mit Ascorbinsäure im wichtigen Entscheidungsbereich von 0-40 mg Ascorbinsäure/dl Harn sehr gut abgestufte Reaktionsfarben von hellgrünblau bis dunkelblauviolett.

Der so hergestellte Test eignet sich auch zum semiquantitativen Nachweis von Ascorbinsäure in Fruchtsäften oder anderen Getränken, wobei vor der endgültigen Bestimmung ggf. mit Wasser verdünnt werden muss. Eine objektive Bewertung der Reaktionsfarben kann mit handelsüblichen Remissionsphotometern vorgenommen werden. In der folgenden Tabelle sind die mit dem Spectralphotometer DMR 21 mit Remissionsaufsatz ZR 21 der Firma Zeiss gemessenen Remissionswerte den mit dem Auge erkennbaren Verfärbungen gegenübergestellt.

| Konzentration der Ascorbinsäure mg % | % Remission bei $\lambda$ 640 nm | Reaktionsfarbe |
|---|---|---|
| 0 | 89 | blass gelb |
| 5 | 36 | hellgrün |
| 10 | 26 | grünblau |
| 20 | 19 | blau |
| 40 | 12 | blauviolett |
| 100 | 8 | dunkelblau-violett |

*Beispiel 2*

6,5 g Natriumsalz der 2,18-Phosphormolybdänsäure, 4 g Natriumchlorat und 45 g Äpfelsäure oder Weinsäure werden in Wasser gelöst und mit 10 n Natronlauge auf pH-Wert zwischen 2,5 und 5,0 eingestellt. Die Weiterverarbeitung zu einem Test geschieht, wie unter Beispiel 1 beschrieben. Die Funktion und Eigenschaften der Teststreifen entsprechen denen des im Beispiel 1 beschriebenen Produkts.

*Beispiel 3*

Ein Reagenzpapier gemäss Beispiel 1 oder Beispiel 2 wird zusammen mit Reagenzpapieren zum Nachweis von Nitrit, pH-Wert, Glucose und Ketonkörpern, die nach bekannten Verfahren hergestellt wurden, durch Einnetzen zu Mehrfachteststreifen verarbeitet. Diese Mehrfachteste wurden in verschlossenen Röhren mehrere Wochen gelagert.

Für Vergleichs-Versuche wurden Reagenzpapiere gemäss Beispiel 1 hergestellt, in denen die Zusammensetzung dahingehend geändert wurde, dass statt Citronensäure und Lithiumcitrat die gleichen Mengen Bernsteinsäure bzw. Malonsäure und Lithiumhydroxid zur Einstellung von pH 3,6 eingesetzt wurden. Diese Reagenzpapiere wurden, wie oben beschrieben, zu Mehrfachtesten verarbeitet, und ebenfalls mehrere Wochen gelagert. Prüft man mit einem Mehrfachteststreifen, der den erfindungsgemässen Ascorbinsäuretest enthält, den pH-Wert von z.B. in Harnen, so wird der richtige pH-Wert gefunden. Prüft man mit Mehrfachteststreifen, die ein Ascorbinsäuretestpapier enthalten, das mit Bernsteinsäure oder Malonsäure hergestellt wurde, so wird ein saurer pH-Wert vorgetäuscht.

Der gleiche negative Einfluss auf das pH-Papier ist zu beobachten, wenn man ein Testpapier, welches gemäss DAS 2 309 794 mit Natrium-2-phospho-18-molybdat/Natriumphospho-12-molybdat/Natriumnitrat und Malonsäure imprägniert ist, in Mehrfachteststreifen einarbeitet und diese über mehrere Wochen lagert.

**Patentansprüche**

1. Schnelltest zum Nachweis von Ascorbinsäure, bestehend aus einem saugfähigen Träger, der mit einem Phosphormolybdat und einer organischen Säure imprägniert ist, dadurch gekennzeichnet, dass pro Liter Imprägnierlösung als Phosphormolybdat 3,0 bis 9,0 g pro Liter eines Alkalisalzes der 2,18-Phosphormolybdänsäure und als organische Säure 10,0 bis 90,0 g pro Liter einer aliphatischen Hydroxycarbonsäure verwendet werden und die Imprägnierung zusätzlich noch 3,0 bis 6,0 g pro Liter eines Alkalichlorats enthält und einen pH-Wert von 2,5 bis 5,0 aufweist.

2. Schnelltest nach Anspruch 1, dadurch gekennzeichnet, dass als Hydroxycarbonsäure Äpfelsäure, Citronensäure oder Weinsäure oder ein Gemisch dieser Säuren mit ihren Alkalisalzen enthalten ist.

3. Verfahren zur Herstellung von Schnelltesten gemäss Anspruch 1, dadurch gekennzeichnet, dass man einen saugfähigen Träger mit einer wässrigen Lösung von

3,0-9,0 g/l Alkalisalz der 2,18-Phosphormolybdänsäure

10,0-90,0 g/l Hydroxycarbonsäure und ihrem Alkalisalz

3,0-6,0 g/l Alkalichlorat

und so viel Alkalihydroxyd, dass ein pH-Wert von 2,6-5,0 eingestellt wird,

tränkt, trocknet und in bekannter Weise weiterverarbeitet.

4. Verfahren nach Anspruch 3, dadurch gekennzeichnet, dass die Lösung zusätzlich noch ein mit Wasser mischbares Lösungsmittel, vorzugsweise einen niederen Alkohol, enthält.

**Claims**

1. Rapid test for the detection of ascorbic acid, consisting of an absorbent carrier which is impregnated with a phosphomolybdate and an oganic acid, characterised in that, per litre of impregnation solution, there are used, as phosphomolybdate, 3.0 to 9.0 g per litre of an alkali metal salt of 2,18-phosphomolybdic acid and, as organic acid, 10.0 to 90.0 g per litre of an aliphatic hydroxycarboxylic acid and the impregnation solution additionally also contains 3.0 to 6.0 g per litre of an alkali metal chlorate and has a pH value of 2.5 to 5.0.

2. Rapid test according to claim 1, characterised in that, as hydroxycarboxylic acid, there is present malic acid, citric acid or tartaric acid and/or a mixture of these acids with their alkali metal salts.

3. Process for the production of rapid tests according to claim 1, characterised in that one impregnates an absorbent carrier with an aqueous solution of

3.0-9.0 g/l. of alkali metal salt of 2,18-phosphomolybdic acid

10.0-90.0 g/l. of hydroxycarboxylic acid and its alkali metal salt

3.0-6.0 g/l. of alkali metal chlorate,

and sufficient alkali metal hydroxide to adjust to a pH value of 2.5-5.0, dries and further works up in known manner.

4. Process according to claim 3, characterised in that the solution additionally also contains a water-miscible solvent, preferably a lower alcohol.

**Revendications**

1. Test rapide pour la mise en évidence de l'acide ascorbique, comprenant un support absorbant imprégné d'un phosphomolybdate et d'un acide organique, caractérisé en ce que dans un litre de solution d'imprégnation la teneur en phosphomolybdate est obtenue au moyen d'une quantité comprise entre 3,0 et 9,0 g par litre d'un sel alcalin de l'acide 2,18-phosphomolybdique et la teneur en acide organique au moyen d'une quantité comprise entre 10,0 et 90,0 g par litre d'un acide hydroxycarboxylique aliphatique et en ce que la solution d'imprégnation contient additionnellement encore entre 3,0 et 6,0 g par litre d'un chlorate alcalin et présente un pH entre 2,5 et 5,0.

2. Test rapide selon la revendication 1, caractérisé en ce qu'il contient comme acide hydroxycarboxylique l'acide malique, l'acide citrique ou l'acide tartrique, ou un mélange de ces acides avec leurs sels alcalins.

3. Procédé pour la fabrication de tests rapides selon la revendication 1, caractérisé en ce qu'on imprègne un support absorbant d'une solution aqueuse contenant:

entre 3,0 et 9,0 g/l de sel alcalin d'acide 2,18-phosphomolybdique

entre 10,0 et 90,0 g/l d'acide hydroxycarboxylique et de son sel alcalin

entre 3,0 et 6,0 g/l de chlorate alcalin

et une quantité d'hydroxyde alcalin suffisante pour ajuster le pH entre 2,5 et 5,0,

on le sèche et on l'élabore de façon connue.

4. Procédé selon la revendication 3, caractérisé en ce que la solution contient en outre encore un solvant miscible à l'eau, de préférence un alcool inférieur.